# EUROPÄISCHE PATENTSCHRIFT

(11) **EP 3 736 258 B1**
(45) Veröffentlichungstag und Bekanntmachung des Hinweises auf die Patenterteilung: **01.11.2023**
(21) Anmeldenummer: 19200445.5
(22) Anmeldetag: 30.09.2019
(51) Int. Cl.: C07C 45/50, C07C 47/02, B01J 31/02, B01J 31/18, B01J 37/02

(54) **VERFAHREN ZUR HYDROFORMYLIERUNG VON KURZKETTIGEN OLEFINEN IN ALKANREICHEN ABGASSTRÖMEN**
PROCESS FOR HYDROFORMYLATION OF SHORT-CHAIN OLEFINS IN ALKANE-RICH EXHAUST GAS STREAMS
PROCÉDÉ D'HYDROFORMYLATION D'OLÉFINES À CHAÎNE COURTE DANS LES FLUX DE GAZ D'ÉCHAPPEMENT RICHES EN ALCANES

(30) Priorität: 05.10.2018 EP 18198789
(43) Veröffentlichungstag der Anmeldung: 11.11.2020
(73) Patentinhaber: Evonik Operations GmbH, 45128 Essen (DE)
(72) Erfinder: Haßelberg, Jennifer, 44139 Dortmund (DE); Franke, Robert, 45772 Marl (DE); Stenger, Frank, 63755 Alzenau (DE); Kreis, Peter, 44227 Dortmund (DE); Hecht, Corinna, 45721 Haltern am See (DE); Kristen, Marc Oliver, 45721 Haltern am See (DE)
(74) Vertreter: Evonik Patent Association

(56) Entgegenhaltungen:
- WO-A1-2015/086634
- DE-A1- 2 055 539
- DE-A1-102014 207 246
- ALBERTUS J. SANDEE ET AL: "ROTACAT: A Rotating Device Containing a Designed Catalyst for Highly Selective Hydroformylation", ADVANCED SYNTHESIS & CATALYSIS, Bd. 343, Nr. 2, 26. Februar 2001 (2001-02-26), Seiten 201-206, XP055576100, DE ISSN: 1615-4150, DOI: 10.1002/1615-4169(20010226)343:2<201::AID- ADSC201>3.0.CO;2-R

## Beschreibung

Das Projekt, das zu dieser Patentanmeldung führte, wurde im Rahmen der Fördervereinbarung Nr. 680395 aus dem Forschungs- und Innovationsprogramm Horizon 2020 der Europäischen Union finanziert.

Die vorliegende Erfindung betrifft ein Verfahren zur Hydroformylierung von kurzkettigen Olefinen, insbesondere C2- bis C5-Olefinen, bei dem das Katalysatorssystem in dem zweiten Hydroformylierungsschritt heterogenisiert auf einem Support aus einem porösen keramischen Material vorliegt, sowie Anlagen zur Durchführung dieses Verfahrens.

Die Hydroformylierung ist mit einer jährlichen globalen Produktionskapazität von mehreren Millionen Tonnen eine der bedeutendsten Reaktionen in der großtechnischen Chemie. Dabei werden Alkene (Olefine) mit einer Mischung aus Kohlenmonoxid und Wasserstoff (auch: Synthesegas oder Syngas) unter Verwendung eines Katalysators zu Aldehyden umgewandelt, die wichtige und wertvolle Zwischenprodukte bei der Herstellung von chemischen Massenprodukten wie Alkoholen, Estern oder Weichmachern sind.

Die Hydroformylierung wird im großtechnischen Maßstab ausschließlich homogen katalysiert durchgeführt. Die löslichen Übergangsmetallkatalysatorsysteme basieren üblicherweise auf Cobalt oder Rhodium, welches oft mit Phosphor-haltigen Liganden, bspw. Phosphinen oder Phosphiten, für die Hydroformylierung von eher kurzkettigen Olefinen eingesetzt wird.

Die Probleme bei den bekannten Verfahren sind vielfältig, die insbesondere damit verknüpft sind, dass sowohl Rhodium als auch Cobalt und deren Verbindungen vergleichsweise teuer sind. Es wird ein hoher energetischer und verfahrenstechnischer Aufwand betrieben, um Katalysatorverluste während des Hydroformylierungsprozesses weitestgehend zu vermeiden, beispielsweise durch teils sehr aufwändige Katalysatorrecyclingschritte. Zudem werden Produktaufreinigungsschritte aufwendiger, um sicherzustellen, dass möglichst keine Katalysatorrückstände im Produkt verbleiben.

Weitere Probleme bei den bekannten homogen katalysierten Verfahren sind die Stabilität der Liganden, die die Bedingungen der Hydroformylierung, wie Temperatur, Druck, pH-Wert, usw., überstehen müssen, und der Verbrauch von dem verwendeten Lösemittel während des Prozesses, der durch Nachdosieren ausgeglichen werden muss.

Um die vorgenannten Probleme bei der homogen katalysierten Hydroformylierung zu umgehen, sind Hydroformylierungsverfahren entwickelt worden, bei denen das Katalysatorsystem heterogenisiert wird, insbesondere durch Immobilisierung auf einem Trägermaterial (vgl. einleitende Diskussion in der WO 2015/028284 A1). Die Begriffe Heterogenisierung und Immobilisierung sind demnach so zu verstehen, dass der Katalysator durch Ausbildung eines dünnen Flüssigkeitsfilms mithilfe einer ionischen Flüssigkeit auf der Oberfläche und/oder in den Poren eines festen Trägermaterials immobilisiert wird und keine Reaktionslösung im klassischen Sinne vorliegt, in der der Katalysator homogen gelöst ist.

In der DE 2 055 539 wird ein solches Hydroformylierungsverfahren beschrieben, bei dem eine Hydridocarbonyl-bis-(tri-subst.-phosphin-, -arsin- oder -stibin)-rhodium-Verbindung als Katalysator auf einem festen Träger eingesetzt wird und die Reaktion in der Gasphase erfolgt.

Im Hinblick auf die Immobilisierung bzw. Heterogenisierung offenbart die bereits erwähnte WO 2015/028284 A1 sogenannte SILP-Systeme (SILP = Supported lonic Liquid Phase), bei der das Katalysatorsystem mit Rhodium, Iridium oder Cobalt als Zentralatom insbesondere auf einem porösen Siliciumdioxid-Träger unter Verwendung einer ionischen Flüssigkeit immobilisiert wird.

In der WO 2015/086634 A1 wird ein zweistufiges Verfahren zur Hydroformylierung offenbart. In der primären Reaktionszone wird ein homogen-katalysierte Hydroformylierung durchgeführt und ein aldehydhaltiges Kreisgas entnommen. Das Kreisgas wird einer Membrantrennung zugeführt und das erhaltene Permeat zu einer sekundären Reaktionszone geleitet, wo eine zweite Hydroformylierung mit einem SILP-System stattfindet.

Das Problem bei den bekannten SILP-Systemen ist jedoch, dass nach einer gewissen Laufzeit eine deutliche Abnahme der Katalysatoraktivität und damit eine Verringerung des Umsatzes beobachtet werden kann. Dies kann auf verschiedene Effekte zurückzuführen sein, beispielsweise eine Kondensation der Produkte in den Poren und entsprechende Folgereaktionen wie Aldolkondensationen, oder die Bildung von Wasser, die zu einer Desaktivierung der Liganden führen kann, die Bildung von Nebenprodukten und/oder die Flutung der Poren, wodurch der Katalysator ausgetragen werden kann.

Die Aufgabe der vorliegenden Erfindung bestand deshalb darin, ein Verfahren zur Herstellung von Aldehyden durch Hydroformylierung bereitzustellen, welches die vorgenannten Probleme nicht aufweist und insbesondere zu einer Erhöhung des Umsatzes sowie der Lebenszeit des Katalysators führt.

Gelöst wird diese Aufgabe gemäß Anspruch 1 dadurch, dass die Hydroformylierung in zwei Schritten erfolgt, wobei in dem zweiten Hydroformylierungsschritt ein Katalysator verwendet wird, bei dem das Katalysatorsystem heterogenisiert auf einem Support aus einem porösen keramischen Material vorliegt.

Ein Gegenstand der vorliegenden Erfindung ist somit ein Verfahren zur Herstellung von Aldehyden durch Hydroformylierung eines ersten Einsatzgemisches, welches C2- bis C5-Olefine als Edukte umfasst, wobei die Hydroformylierung mindestens zwei Hydroformylierungsschritte umfasst, dadurch gekennzeichnet, dass

in dem ersten Hydroformylierungsschritt das Einsatzgemisch mit Synthesegas in Gegenwart eines ersten Katalysatorsystems in einer ersten Reaktionszone hydroformyliert wird,
aus der ersten Reaktionszone kontinuierlich ein gasförmiger Austrag entnommen wird, der mindestens einen Teil der entstandenen Produktaldehyde, mindestens einen Teil der nicht umgesetzten Olefine und Synthesegas enthält,
der gasförmige Austrag aus der ersten Hydroformylierung einem ersten Stofftrennungsschritt unterworfen wird, bei der der gasförmige Austrag in eine Synthesegas-reiche Phase, in eine Synthesegas-arme-Phase und optional einen Purgegasstrom getrennt wird, wobei die Synthesegas-reiche Phase zur ersten Reaktionszone zurückgeführt wird und die Synthesegas-arme Phase einem zweiten Stofftrennungsschritt unterworfen wird, bei der die Synthesegas-arme Phase in mindestens eine an nicht umgesetzten Olefinen-reiche Phase und mindestens eine Produktaldehyd-reiche Phase getrennt wird;
die nach Durchlaufen des zweiten Stofftrennungsschritts erhaltene an nicht umgesetzten Olefinen-reiche Phase und optional zumindest ein Teil des Purgegasstroms als zweites Einsatzgemisch zum zweiten Hydroformylierungsschritt geleitet wird; und
in dem zweiten Hydroformylierungsschritt ein zweites Einsatzgemisch in gasförmigem Zustand mit Synthesegas in Gegenwart eines zweiten Katalysatorsystems, welches ein Metall aus der 8. oder 9. Gruppe des Periodensystems der Elemente, mindestens einen organischen Phosphor-enthaltenden Liganden, einen Stabilisator und optional eine ionische Flüssigkeit umfasst, in einer zweiten Reaktionszone einer weiteren Hydroformylierung unterworfen wird, wobei das zweite Einsatzgemisch und das Synthesegas über einen Support aus einem porösen keramischen Material geleitet werden, auf dem das Katalysatorsystem heterogenisiert vorliegt,
wobei der Support ein Monolith, das heißt ein Block aus einem keramischen Material ist, auf den ein Washcoat aus, bezogen auf das keramische Material des Supports, dem gleichen oder einem anderen keramischen Material aufgetragen wird.

Als erstes Einsatzgemisch können alle Gemische eingesetzt werden, die C2- bis C5-Olefine, insbesondere Ethen, Propen, 1-Buten, 2-Buten, 1-Penten oder 2-Penten, als Edukte umfassen. Die Menge an Olefinen in den Einsatzgemischen sollte verständlicherweise hoch genug sein, um eine Hydroformylierungsreaktion wirtschaftlich betreiben zu können. Dazu gehören insbesondere technische Gemische der petrochemischen Industrie, wie beispielsweise Raffinatströme (Raffinat I, II oder III) oder Rohbutan. Rohbutan umfasst gemäß der vorliegenden Erfindung 5 bis 40 Gew.-% Butene, vorzugsweise 20 bis 40 Gew.-% Butene (die Butene setzen sich zusammen aus 1 bis 20 Gew.-% 1-Buten und 80 bis 99 Gew.-% 2-Buten) und 60 bis 95 Gew.-% Butane, vorzugsweise 60 bis 80 Gew.-% Butane.

Der erste Hydroformylierungsschritt in der ersten Reaktionszone entspricht insbesondere einer homogen katalysierten Hydroformylierung, bei der das erste Katalysatorsystem (vollständig) gelöst in der flüssigen Phase des Reaktionsgemisches vorliegt. Das Katalysatorsystem der ersten Hydroformylierung umfasst vorzugsweise ein Übergangsmetall aus der 8. oder 9. Gruppe des Periodensystems der Elemente (PSE) und mindestens einen organischen Phosphor-haltigen Liganden.

Der organische Phosphor-enthaltende Ligand für das erste Katalysatorsystem weist vorzugsweise die allgemeine Formel (VI)

R'-A-R"-A-R‴ (VI)

wobei R', R" und R‴ jeweils organische Reste sind, mit der Maßgabe das R' und R‴ nicht identisch sind, und beide A jeweils eine brückende -O-P(-O)₂-Gruppe sind, wobei zwei der drei Sauerstoffatome -O-jeweils an Rest R' und den Rest R‴ gebunden sind. Die organischen Reste R', R" und R‴ enthalten vorzugsweise keine endständige Trialkoxysilangruppe.

In einer bevorzugten Ausführungsform sind R', R" und R‴ in der Verbindung der Formel (VI), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R‴ nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe.

Als Übergangsmetall können insbesondere Eisen, Ruthenium, Iridium, Cobalt oder Rhodium, vorzugsweise Cobalt oder Rhodium eingesetzt werden. Als katalytische aktive Spezies werden üblicherweise (Liganden)-Carbonyl-Komplexe der Metallatome diskutiert, die sich unter den Drücken und Temperaturen im flüssigen Reaktionsgemisch bilden.

Die erste Hydroformylierung kann insbesondere in Anwesenheit eines Lösemittels durchgeführt werden, wobei das Lösemittel mit dem Hydroformylierungsverfahren kompatibel sein sollte. Als Lösemittel können die dem Fachmann bekannten Lösemittel für die klassische Hydroformylierung verwendet werden, beispielsweise Alkane, aromatische Kohlenwasserstoffe, Wasser, Ether, Ester, Ketone, Alkohole und die Reaktions- oder Nebenprodukte der Hydroformylierung wie Aldehyde und Kondensationsprodukte der Aldehyde.

Weiterhin kann die erste Hydroformylierung bei einem Druck von 10 bis 400 bar, vorzugsweise 15 bis 250 bar durchgeführt werden. Die Temperatur bei der Hydroformylierung kann 70 bis 250 °C, vorzugsweise 100 bis 200 °C betragen. Die Druck- und Temperaturbedingungen entsprechend grundsätzlich den typischen Bedingungen für klassische homogen katalysierte Hydroformylierungen. Wichtig ist lediglich, dass nach der ersten Hydroformylierung oder nach den Stofftrennungsschritten eine gasförmige Phase entsteht, die zumindest einen Teil der nicht umgesetzten Olefine umfasst und die zur zweiten Hydroformylierung geführt werden kann.

Die erste Reaktionszone umfasst mindestens einen Reaktor, in dem der erste Hydroformylierungsschritt durchgeführt wird. Es sind natürlich auch Ausführungsformen denkbar, bei der mehrere gleichzeitig betriebene Reaktoren parallel geschaltet oder in Reihe geschaltet vorliegen. Als Reaktor für den ersten Hydroformylierungsschritt kommen alle dem Fachmann bekannten und für die Hydroformylierung geeigneten Reaktortypen, insbesondere Gas-Flüssig-Reaktoren, in Betracht. Geeignete Reaktortypen sind insbesondere Rührkesselreaktoren, Blasensäulenreaktoren, kaskadierte Blasensäulenreaktoren, Jetloopreaktoren oder Schlaufenreaktoren.

Aus der ersten Reaktionszone wird nach dem ersten Hydroformylierungsschritt kontinuierlich ein gasförmiger Austrag entnommen, der mindestens einen Teil der entstandenen Produktaldehyde, mindestens einen Teil der nicht umgesetzten Olefine und Synthesegas enthält. Der gasförmige Austrag kann weitere Stoffe, beispielsweise im ersten Einsatzgemisch bereits enthaltene Alkane oder bei der ersten Hydroformylierung entstandene Hochsieder, beispielsweise Acetale, Aldole, Halbacetale oder anderer Nebenprodukte, enthalten.

Der gasförmige Austrag durchläuft nach der ersten Hydroformylierung zunächst einen ersten Stofftrennungsschritt, bei dem der gasförmige Austrag in mindestens eine Synthesegas-reiche Phase, die neben Synthesegas auch inerte Stoffe, insbesondere Alkane, und geringe Anteile an nicht umgesetzten Olefinen oder Produktaldehyde enthalten kann, und mindestens eine Synthesegas-arme Phase, die zumindest Produktaldehyde und nicht umgesetzte Olefine enthält, getrennt wird.

Bei der ersten Stofftrennung kann neben den beiden genannten Phasen auch ein Purgegasstrom abgetrennt werden, der eine ähnliche oder eine zur Synthesegas-reichen Phase identische Zusammensetzung aufweist. Der Purgegasstrom kann dadurch erzeugt werden, dass der Strom der Synthesegas-reichen Phase nach erfolgter erster Stofftrennung aufgeteilt wird, wobei der Purgegasstrom insbesondere einen kleineren Volumenstrom aufweist als der Strom der Synthesegas-reichen Phase.

Das Auftrennen und die Abnahme eines Purgegasstroms kann vorteilhaft sein, weil dadurch verhindert werden kann, dass sich Verunreinigungen des Synthesegases, beispielsweise N₂, oder inerte Stoffe, die im Einsatzgemisch vorhanden sind (beispielsweise Alkane) in dem geschlossenen Hydroformylierungssystem anreichern. Der Purgegasstrom wird dann nicht zur ersten Reaktionszone zurückgefahren sondern den nachfolgenden Prozessen (bspw. zweite Stofftrennung) zugeführt, wodurch die genannten Verunreinigungen und Inertverbindungen nicht zwangsläufig im System verbleiben und sich anreichern, sondern ausgeschleust werden können.

Die durch die erste Stofftrennung erhaltene Synthesegas-reiche Phase wird zu ersten Reaktionszone zurückgeführt. Dadurch wird das Synthesegas im Kreis gefahren und steht der Hydroformylierung in der ersten Reaktionszone zur Verfügung. Der optional gebildete Purgegasstrom kann zur zweiten Stofftrennung und/oder ohne Durchlaufen der zweiten Stofftrennung direkt zur zweiten Reaktionszone geführt werden. Durch diese Verfahrensführung kann das für den zweiten Hydroformylierungsschritt benötige Synthesegas zumindest teilweise aus direkt aus dem Verfahren bereitgestellt werden und nicht ausschließlich extern zugeführt werden. Dem zweiten Hydroformylierungsschritt kann optional zusätzliches Synthesegas zugeführt werden.

Die Synthesegas-arme-Phase wird zur zweiten Stofftrennung geleitet, bei der die Synthesegas-arme-Phase in mindestens eine an nicht umgesetzten Olefinen-reiche Phase, die neben nicht umgesetzten Olefinen auch Synthesegas und inerte Stoffe, insbesondere Alkane, umfassen kann, und mindestens eine Produktaldehyd-reiche Phase, die neben dem Produktaldehyd auch nicht umgesetzte Olefine und inerte Stoffe, insbesondere Alkane, umfassen kann, getrennt wird.

Der erste und zweite Stofftrennungsschritt kann mit bekannten Stofftrennungsverfahren, wie Kondensation, Destillation, Zentrifugation, Nanofiltration oder Kombination von mehreren davon, vorzugsweise Kondensation oder Destillation durchgeführt werden. In einer bevorzugten Ausführungsform werden der erste und der zweite Stofftrennungsschritt nicht identisch, sondern wird jeweils mit einem unterschiedlichen Stofftrennungsverfahren durchgeführt. In einer ganz bevorzugten Ausführungsform der vorliegenden Erfindung ist der erste Stofftrennungsschritt eine Kondensation und der zweite Stofftrennungsschritt eine Destillation.

Die an nicht umgesetzten Olefinen-reiche Phase wird als zweites Einsatzgemisch für die zweite Hydroformylierung verwendet. Optional kann auch zumindest ein Teil des Purgegasstroms mit der an nicht umgesetzten Olefinen-reiche Phase kombiniert werden und zum zweiten Hydroformylierungsschritt gefahren werden. Das zweite Einsatzgemisch muss in gasförmigem Zustand vorliegen. Dies kann beispielsweise dadurch sichergestellt werden, dass die als zweites Einsatzgemisch eingesetzten Phasen vorher einen Wärmetauscher durchlaufen, um sicherzustellen, dass das gesamte Einsatzgemisch in der Gasphase vorliegt.

Es hat sich gezeigt, dass Olefine, die im ersten Hydroformylierungsschritt nicht umgesetzt worden sind, im zweiten Hydroformylierungsschritt durch die Verwendung von auf porösen keramischen Materialien heterogenisierten Katalysatorssystemen noch zum gewünschten Produktaldehyd umgesetzt werden können. Die Ausgestaltung des Verfahrens, die eine homogen katalysierte Hydroformylierung in flüssiger Phase mit einer weiteren Hydroformylierung in der Gasphase mit einem heterogenisierten Katalysatorsystem kombiniert, ist auch deshalb vorteilhaft, weil die Edukte in gasförmigem Zustand zum zweiten Hydroformylierungsschritt zugeführt werden können. Dadurch können ein solcher zweiter Hydroformylierungsschritt und die entsprechende Reaktor- bzw. Verfahrenstechnik vergleichsweise einfach in bestehende Anlagen stromabwärts eines bereits bestehenden Flüssigphasen-Hydroformylierungsreaktors integriert werden.

Das erfindungsgemäße Verfahren betrifft insbesondere technische Hydroformylierungsverfahren, die nach dem gas-recycle-Prinzip (auch Kreisgasverfahren oder stripping reactor process) betrieben werden. Dabei werden Hydroformylierungsprodukte mit überschüssigen Synthesegas gasförmig aus dem Reaktor ausgetragen, wobei üblicherweise auch Nebenprodukte und/oder nicht umgesetzte Einsatzolefine mitgerissen werden.

Das im zweiten Hydroformylierungsschritt eingesetzte zweite Katalysatorsystem umfasst vorzugsweise ein Übergangsmetall aus der 8. oder 9. Gruppe des Periodensystems der Elemente, insbesondere Eisen, Ruthenium, Iridium, Cobalt oder Rhodium, besonders bevorzugt Cobalt und Rhodium, mindestens einen organischen Phosphor-enthaltenden Liganden, einen Stabilisator und optional eine ionische Flüssigkeit.

Der Stabilisator ist vorzugsweise eine organische Aminverbindung, besonders bevorzugt eine organische Aminverbindung, die mindestens eine 2,2,6,6-Tetramethylpiperidineinheit nach Formel (I) enthält:

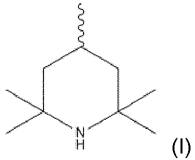

In einer besonders bevorzugten Ausführungsform der vorliegenden Erfindung ist der Stabilisator ausgewählt aus der Gruppe, bestehend aus den Verbindungen der nachfolgenden Formeln (I.1), (I.2), (I.3), (I.4), (I.5), (I.6), (I.7) und (I.8). 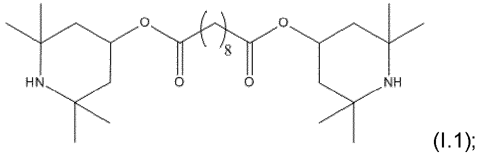 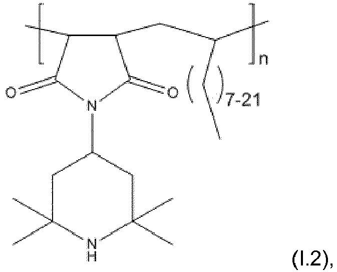
wobei n einer ganzen Zahl von 1 bis 20 entspricht; 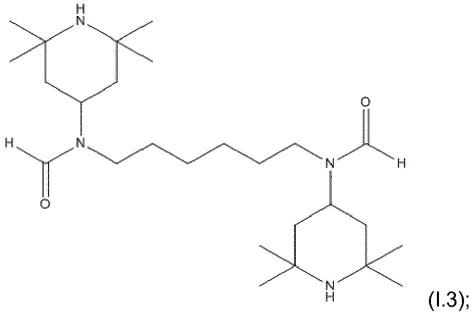 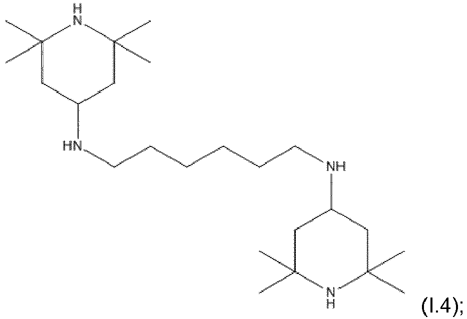 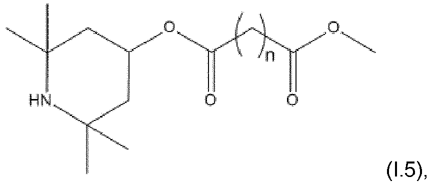
wobei n einer ganzen Zahl von 1 bis 12 entspricht; 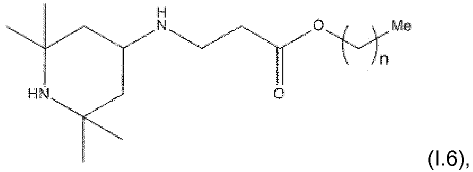
wobei n einer ganzen Zahl von 1 bis 17 entspricht; 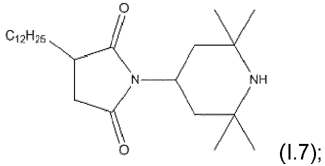 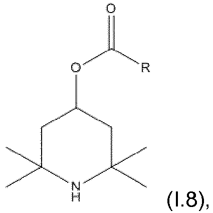
wobei R einer C6- bis C20-Alkylgruppe entspricht.

Die optional vorliegende ionische Flüssigkeit im Sinne der vorliegenden Erfindung ist eine nahezu wasserfrei (Wassergehalt < 1,5 Gew.-% bezogen auf die gesamte ionische Flüssigkeit) Flüssigkeit, die bei Normaldruck (1,01325 bar) und vorzugsweise bei 25 °C flüssig ist. Die ionische Flüssigkeit besteht vorzugsweise zu mehr als 98 Gew.-% aus Ionen.

In einer bevorzugten Ausführungsform wird das Anion der ionischen Flüssigkeit ausgewählt aus der Gruppe, bestehend aus Tetrafluoroborat [BF4]⁻; Hexafluorophosphat [PF6]⁻; Dicyanamid [N(CN)₂]⁻; Bis(trifluoromethylsulfonyl)imid [NTf₂]⁻; Tricyanomethid [C(CN)₃]⁻; Tetracyanoborat [B(CN)₄]⁻; Halogeniden, insbesondere Cl⁻, Br⁻, F⁻, I⁻; Hexafluoroantimonat [SbF₆]⁻; Hexafluoroarsenat [AsF₆]⁻; Sulfat [SO₄]²⁻; Tosylat [C₇H₇SO₃]⁻; Triflat CF₃SO₃⁻; Nonaflat [C₄F₉SO₃]⁻; Tris-(Pentafluoroethyl)-trifluorophosphat [PF₃(C₂F₅)₃]⁻; Thiocyanat [SCN]⁻; Carbonat [CO₃]²⁻; [RA-COO]⁻; [RA-SO₃]⁻; [RA-SO₄]⁻; [RAPO₄RB]- und [(RA-SO₂)₂N]⁻, wobei RA und RB gleich oder ungleich voneinander sein können und jeweils eine lineare oder verzweigte aliphatischer oder alicyclischer Alkylgruppe mit 1 bis 12 Kohlenstofatomen, eine Perfluoralkylgruppe oder eine C5-C18-substituierte Arylgruppe ist, die durch ein oder mehrere Halogenatome substituiert ein kann.

Das Kation der ionischen Flüssigkeit wird vorzugsweise ausgewählt aus der Gruppe, bestehend aus quaternäre Ammonium-Kationen der allgemeinen Formel [NR¹R²R³R⁴]⁺ wobei R¹, R², R³, R⁴ jeweils unabhängig voneinander eine C1-C8-Alkylgruppe darstellen; Phosphonium-Kationen der allgemeinen Formel [PR¹R²R³R⁴]⁺ wobei R¹, R², R³, R⁴ jeweils unabhängig voneinander eine C1-C8-Alkylgruppe darstellen; Imidazolium-Kationen der allgemeinen Formel (II) 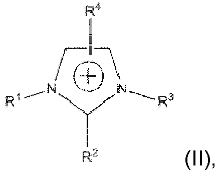
wobei R¹, R², R³ und R⁴ jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Pyridinium-Kationen der allgemeinen Formel (III) 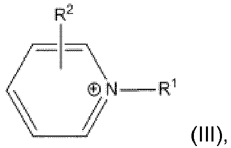
wobei R¹ und R² jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Pyrazolium-Kationen der allgemeinen Formel (IV) 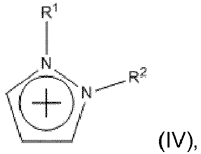
wobei R¹ und R² jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen;
Triazolium-Kationen der allgemeinen Formel (V) 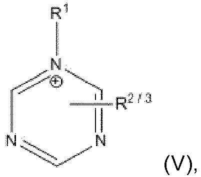
wobei R¹ und R² und/oder R³ jeweils unabhängig voneinander H oder eine C1 bis C8-Alkylgruppe, eine C1 bis C6-Alkoxygruppe, eine optional substituierte C1 bis C6-Aminoalkylgruppe oder eine optional substituierte C5 bis C12-Arylgruppe darstellen.

In einer bevorzugten Ausführungsform ist das Kation der ionischen Flüssigkeit ein Imidazolium-Kation nach der vorgenannten allgemeinen Formel (II) mit entsprechender Definition der Reste R¹ bis R⁴. In einer besonders bevorzugten Ausführungsform ist die ionische Flüssigkeit ausgewählt aus der Gruppe, bestehend aus 1-Ethyl-3-methylimidazolium bis(trifluoromethylsulfonyl)imid, 1-Butyl-3-methylimidazolium hexafluorophosphat, 1-Butyl-3-methylimidazolium tetrafluoroborat, 1-Butyl-3-methylimidazolium bis(trifluoromethylsulfonyl)imid, 1-Ethyl-3-methylimidazolium ethylsulfat, Trioctyl-Methylammonium bis(trifluoromethylsulfonyl)imid und 1-Butyl-3-methylimidazolium octylsulfat.

Die optional vorhandene ionische Flüssigkeit dient in dem erfindungsgemäßen Katalysatorsystem als Trägerlösung für den Übergangsmetallkatalysator mit Liganden und den Stabilisator. Dabei ist es wichtig, dass die ionische Flüssigkeit die Reaktanden (Einsatzolefine und Synthesegas) in ausreichendem Maße aufnehmen, d. h. lösen, kann und einen vergleichsweise niedrigen Dampfdruck aufweist, damit das Katalysatorsystem auch bei hohen Temperaturen als Flüssigkeitsreservoir vorliegt. Es konnte jedoch überraschenderweise herausgefunden werden, dass auch der Stabilisator einen stabilen Flüssigkeitsfilm in den Poren des Supports ausbilden kann und damit in der Lage ist, die ionische Flüssigkeit teilweise oder vollständig zu ersetzen.

Für alle filmbildenden Komponenten, d. h. in diesem Fall die ionische Flüssigkeit und/oder der Stabilisator, sollte die Gaslöslichkeit für die Reaktanden besser sein als die Gaslöslichkeit der Produkte. Bereits dadurch kann eine partielle Stofftrennung zwischen eingesetzten Eduktolefinen und gebildeten Produktaldehyden erreicht werden. Grundsätzlich wären dafür auch andere filmbildende Substanzen denkbar, allerdings ist darauf zu achten, dass es nicht zu einer erhöhten Hochsiederbildung kommt und/oder die Nachführung der Eduktolefine eingeschränkt wird.

Der organische Phosphor-enthaltende Ligand für das zweite Katalysatorsystem weist vorzugsweise die allgemeine Formel (VI)

R'-A-R"-A-R‴ (VI)

wobei R', R" und R‴ jeweils organische Reste sind, mit der Maßgabe das R' und R‴ nicht identisch sind, und beide A jeweils eine brückende -O-P(-O)₂-Gruppe sind, wobei zwei der drei Sauerstoffatome -O-jeweils an Rest R' und den Rest R‴ gebunden sind. Die organischen Reste R', R" und R‴ enthalten vorzugsweise keine endständige Trialkoxysilangruppe.

In einer bevorzugten Ausführungsform sind R', R" und R‴ in der Verbindung der Formel (VI), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R‴ nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe.

Das vorgenannte zweite Katalysatorsystem liegt erfindungsgemäß heterogenisiert auf einem Support aus einem porösen keramischen Material vor. Im Sinne der vorliegenden Erfindung ist der Ausdruck "heterogenisiert auf einem Support" so zu verstehen, dass das Katalysatorsystem durch Ausbildung eines dünnen, festen oder flüssigen Films mithilfe des Stabilisators und/oder optional der ionischen Flüssigkeit auf der inneren und/oder äußeren Oberfläche eines festen Trägermaterials immobilisiert wird. Der Film kann auch bei Raumtemperatur fest und bei Reaktionsbedingungen flüssig sein.

Die innere Oberfläche des festen Trägermaterials umfasst insbesondere die innere Oberfläche der Poren und/oder Kanäle. Immobilisierung umfasst begrifflich sowohl den Fall, dass das Katalysatorsystem und/oder die katalytisch aktive Spezies gelöst in dem festen oder flüssigen Film vorliegen, als auch die Fälle, dass der Stabilisator als Haftvermittler wirkt oder dass das Katalysatorsystem auf der Oberfläche adsorbiert wird, nicht jedoch chemisch bzw. kovalent gebunden auf der Oberfläche vorliegt.

Es liegt erfindungsgemäß also keine Reaktionslösung im klassischen Sinne vor, in der der Katalysator homogen gelöst ist, sondern das Katalysatorsystem befindet sich dispers verteilt auf der Oberfläche und/oder in den Poren des Supports.

Das poröse keramische Material ist vorzugsweise ausgewählt aus der Gruppe, bestehend aus einer Silikatkeramik, einer oxidischen Keramik, einer nitridischen Keramik, einer carbidischen Keramik, einer silicidischen Keramik und Mischungen davon.

Die Silikatkeramik ist vorzugsweise ausgewählt aus Alumosilikat, Magnesiumsilikat und Mischungen davon, wie z. B. Bentonit. Die oxidische Keramik ist vorzugsweise ausgewählt aus γ-Aluminiumoxid, α-Aluminiumoxid, Titandioxid, Beryliumoxid, Zirkoniumoxid, Aluminiumtitanat, Bariumtitanat, Zinkoxid, Eisenoxide (Ferrite) und Mischungen davon. Die nitridische Keramik ist vorzugsweise ausgewählt aus Siliciumnitrid, Bornitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung, auf den das zweite Katalysatorsystem aufgebracht wird, besteht vorzugsweise aus einer carbidischen Keramik.

Der Support ist ein Monolith, das heißt der Support aus dem porösen keramischen Material besteht aus einem Block (ein dreidimensionales Objekt) aus einem keramischen Material. Der Block kann sowohl einstückig ausgebildet sein als auch aus mehreren, also mindestens zwei Einzelteilen bestehen, die zu dem Block zusammengefügt werden können und/oder miteinander fest oder lösbar verbunden sind. Der Support ist aber insbesondere kein Granulat, welches als Katalysatorschüttung in Festbettreaktoren eingesetzt werden kann.

Der Support aus dem porösen keramischen Material ist vorzugsweise ein sich dreidimensional erstreckendes Bauteil, welches in seinem Querschnitt grundsätzlich beliebige geometrische Formen, beispielsweise rund, eckig, quadratisch o. ä., aufweisen kann. Das sich dreidimensional erstreckende Bauteil, welches als Support eingesetzt werden kann, weist in einer bevorzugten Ausführungsform eine Längsrichtung (Richtung der längsten Ausdehnung) in Hauptdurchströmungsrichtung (Richtung in die das Einsatzgemisch und das Synthesegas von Einlass zum Auslass des Reaktors strömen) auf.

Der so geformte Support aus dem porösen keramischen Material weist zumindest einen durchgängigen Kanal in Hauptdurchströmungsrichtung auf. Der oder die Kanäle können jedoch auch so ausgestaltet sein, dass sie nicht komplett durchgängig sind, sondern zu dem Ende, welches dem Einlass des Reaktors gegenüberliegt, einen Abschluss aufweisen bzw. der Kanal zu diesem Ende hin geschlossen ist. Der Support kann auch mindestens zwei oder mehr Kanäle aufweisen. Der Durchmesser der Kanäle kann im Bereich von 0,25 bis 50 mm, vorzugsweise im Bereich von 1 bis 30 mm, weiterhin bevorzugt im Bereich von 1,5 bis 20 mm und besonders bevorzugt im Bereich von 2 bis16 mm liegen. Sind mehrere Kanäle vorhanden, können die Durchmesser der Kanäle gleich oder verschieden voneinander sein. Der Durchmesser der Kanäle ist im Vergleich zu dem oder zu einem der Durchmesser des gesamten Supports insbesondere so zu wählen, dass die mechanische Stabilität nicht beeinträchtigt wird.

Darüber hinaus ist der Support aus dem keramischen Material porös, weist also Poren auf. Das erfindungsgemäße Katalysatorsystem befindet sich insbesondere auch in dem festen oder flüssigen Film in diesen Poren. Der Porendurchmesser liegt vorzugsweise im Bereich von 0,9 nm bis 30 µm, vorzugsweise im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden.

In einer bevorzugten Ausführungsform weist der Support zumindest teilweise durchgängige Poren auf, die sich von der Oberfläche zu den Kanälen hin und/oder von einem Kanal zu dem oder zu den nächstliegenden Kanälen erstrecken. Möglich ist auch das mehrere Poren miteinander verbunden sind und so insgesamt eine einzige durchgängige Pore bilden.

Die Herstellung des Supports aus einem porösen keramischen Material, auf dem das Katalysatorsystem heterogenisiert vorliegt, erfolgt wie nachfolgend beschrieben: Auf den Support aus dem keramischen Material wird zusätzlich ein sogenannter Washcoat aufgetragen, welcher bezogen auf das keramische Material des Supports aus dem gleichen oder einem unterschiedlichen Keramikmaterial, insbesondere ein aus den vorgenannten Keramikmaterialen ausgewähltes Keramikmaterial, vorzugsweise Siliciumoxid. Der Washcoat selber kann porös oder unporös sein, vorzugsweise ist der Waschcoat unporös. Die Partikelgröße des Washcoats beträgt vorzugsweise 5 nm bis 3 µm, vorzugsweise 7 nm bis 700nm. Der Washcoat wird verwendet, um die gewünschte Porengröße einzubringen oder zu generieren und/oder, um die Oberfläche des Supports zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports.

Auf den so erzeugten keramischen Support mit dem aufgebrachten Washcoat wird das Katalysatorsystem aufgebracht. Dazu wird zunächst eine Katalysatorlösung durch Mischen, insbesondere bei Raumtemperatur und Umgebungsdruck, hergestellt, die mindestens einen organischen Phosphor-haltigen Ligand, mindestens einen Metall-Precursor, beispielsweise Chloride, Oxide, Carboxylate des jeweiligen Übergangsmetalls, mindestens einen Stabilisator und mindestens ein Lösemittel umfasst. Optional kann bei der Herstellung des Katalysatorsystems eine ionische Flüssigkeit verwendet werden, die Katalysatorlösung kann aber auch explizit ohne ionische Flüssigkeit angesetzt werden. Die Herstellung der Katalysatorlösung sollte insbesondere in einer inerten Umgebung, bspw. einer Glovebox erfolgen. Inerte Umgebung heißt in diesem Fall eine möglichst Wasser- und Sauerstoff-freie Atmosphäre.

Das Lösemittel kann aus allen Lösemittelklassen gewählt werden (protisch, aprotisch, polar oder unpolar) Voraussetzung für das Lösemittel ist die Löslichkeit von Katalysatorsystem (Ligand, Metall Precursor, Stabilisator und optional die ionische Flüssigkeit) und bevorzugt auch der bei der Hydroformylierung entstehenden Hochsieder. Die Löslichkeit kann innerhalb des Immobilisierungsschrittes durch Aufheizen erhöht werden.

Das Lösemittel ist vorzugsweise aprotisch, polar, wie z. B. Acetonitril und Ethylacetat oder aber auch aprotisch, unpolar wie z. B. THF und Dietehylether. Auch Chlorkohlenwasserstoffe wie z. B. Dichlormethan, können als Lösemittel verwendet werden.

Die so hergestellte Katalysatorlösung wird dann mit dem Support (optional inkl. Washcoat) in Kontakt gebracht, beispielsweise durch Eintauchen (Dip-Coating) oder mittels Befüllen in einem Druckgefäß, beispielsweise direkt im Reaktor (in-situ-Imprägnierung). Sofern das Aufbringen der Katalysatorlösung außerhalb des Reaktors erfolgt, muss der Support nach Abtrennen des Lösemittels natürlich wieder in den Reaktor eingebaut werden. Bevorzugt wird die Katalysatorlösung direkt im Reaktor auf den Support mit dem Washcoat aufgebracht, weil dadurch möglicherweise zeitaufwändige Ein- und Ausbauschritte sowie eine mögliche Kontamination des Katalysators vermieden werden können.

Bei der in-situ-Imprägnierung wird der Reaktor vor dem Befüllen mit einem Inertgas, beispielsweise Edelgase, Alkane oder Stickstoff, gespült. Das Spülen kann bei 1 bis 25 bar durchgeführt werden, vorzugsweise unter leichtem Überdruck von 20 bis 90 mbar, besonders bevorzugt 30 bis 60 mbar über Normaldruck. Der Reaktor kann vor dem Spülen mit Inertgas abgekühlt werden, um zu verhindern, dass das Lösemittel der einzufüllenden Katalysatorlösung sofort verdampft. Weist das Lösemittel jedoch eine Siedetemperatur auf, die größer ist als die Temperatur des Reaktors kann das Abkühlen des Reaktors entfallen.

Nach dem Spülen mit Inertgas kann der vorhandene Druck, beispielsweise über die Druckregelung, abgelassen werden, vorzugweise bis der Reaktor drucklos ist, also Umgebungsdruck (ca. 1 bar) aufweist. Andererseits kann in dem Reaktor, auch ein Vakuum erzeugt werden, beispielsweise mit einer Vakuumpumpe. In einer Ausgestaltung der vorliegenden Erfindung kann der Reaktor, nach dem Ablassen des Drucks oder nach dem Ziehen des Vakuums erneut mit einem Inertgas wie oben beschrieben gespült werden. Dieser Vorgang von Druck ablassen / Vakuum ziehen und erneutem Spülen kann beliebig oft wiederholt werden.

Die Katalysatorlösung wird für das Befüllen des Reaktors in einem Druckgefäß vorgelegt und vorzugsweise mit einem Inertgasüberdruck von 1 bis 25 bar, besonders bevorzugt einem leichten Inertgasüberdruck von 20 bis 90 mbar, vorzugsweise 30 bis 60 mbar über Reaktordruck beaufschlagt. Das Inertgas kann ein Edelgas, ein Alkan, beispielsweise Butan, oder Stickstoff sein. Die Katalysatorlösung wird dann mit dem genannten Überdruck, mit dem das Druckgefäß beaufschlagt ist, insbesondere druckgetrieben in den Reaktor eingefüllt. Der Druck des Druckgefäßes sollte beim Befüllen höher sein als im Reaktor. Dabei können Temperaturen im Bereich von 20 bis 150 °C und ein Druck von 1 bis 25 bar vorliegen.

Eine andere Möglichkeit des Befüllens ist, dass der Reaktor nach dem Spülen mit Inertgas im Vakuum gehalten wird und die Katalysatorlösung durch den Unterdruck in den Reaktor gezogen wird. Dabei sollte für die Herstellung der Katalysatorlösung ein Lösemittel verwendet werden, welches bei dem herrschenden Vakuum bzw. Unterdruck und den herrschenden Temperaturen siedet.

Das Befüllen des Reaktors mit der Katalysatorlösung kann über die normalen Ein- bzw. Ausgänge erfolgen. Flüssigkeitsverteiler oder Düsen innerhalb des Reaktors können für eine gleichmäßige Verteilung der Katalysatorflüssigkeit sorgen, ebenso wie optional vorliegende Druckverlusteinbauten oder Regelungen für die Dosiergeschwindigkeit.

Nach dem Aufbringen des Katalysatorsystems wird das Lösemittel abgetrennt. Dabei wird zunächst die restliche Katalysatorlösung über den Auslass des Reaktors abgelassen. Danach werden im Reaktor verbliebene Lösemittelreste durch Einstellen des Drucks oder Erhöhung der Temperatur verdampft. Die Einstellung des Drucks kann in einer anderen Ausführungsform auch unter gleichzeitiger Erhöhung der Temperatur durchgeführt werden. Die Temperatur kann in Abhängigkeit vom Lösemittel 20 bis 150 °C betragen. Der Druck kann in Abhängigkeit vom Lösemittel auf ein Hochvakuum (10⁻³ bis 10⁻⁷ mbar) eingestellt werden, je nach Lösemittel und Temperatur sind aber auch Überdrücke von einigen mbar bis zu mehreren bar denkbar.

Der Stabilisator und die optional vorhandene ionische Flüssigkeit verbleiben mit dem Katalysator aus dem Übergangsmetall, insbesondere Cobalt oder Rhodium, und dem organischen Phosphor-haltigen Liganden heterogenisiert auf dem Support.

Das Aufbringen des Katalysatorsystems auf den Support kann sowohl direkt im Reaktor (in situ) oder außerhalb des Reaktors erfolgen. Das Aufbringen des Katalysatorsystems wird in einer bevorzugten Ausführungsform der vorliegenden Erfindung direkt im Reaktor, also in situ, aufgebracht. Nach dem Abtrennen des Lösemittels kann der Reaktor sofort eingesetzt werden und mit dem zweiten Einsatzgemisch beschickt werden. Das hat den Vorteil, dass keine zeitaufwändigen Ein- und Ausbauschritte notwendig sind, die einen längeren Ausfall des Reaktors zur Folge hätten. Zudem ist die Größe des Supports dann nicht mehr dadurch limitiert, dass geeignete Räumlichkeiten mit inerten Umgebungen einer bestimmten Größe vorliegen. Die Größe des Supports kann in Abhängigkeit vom Reaktordesign frei gewählt werden. Ein weiteres Problem ist, dass der Support immer unter Luftabschluss transportiert werden muss, was teilweise schwer zu realisieren ist.

Nach erfolgtem Aufbringen des Katalysatorsystems auf den Support und erfolgter Abtrennung des Lösemittels kann die Anlage, insbesondere der Reaktor durch eine zwei- oder mehrstufige Anfahrprozedur hochgefahren werden, also in den Betrieb überführt werden.

Das Ziel der Anfahrprozedur ist eine schonende Aktivierung des Katalysatorsystems und eine Abfedern der maximalen Startaktivität des Katalysators zur Verlängerung der Lebenszeit des Katalysatorsystems. Außerdem soll durch die Anfahrprozedur die Ausbildung einer Flüssigphase verhindert werden, da dies zu einer Desaktivierung, Blockierung und oder zum Auswaschen des Katalysatorsystems führen kann. Besonders beim Anfahren eines frisch hergestellten Katalysatorsystems (auf dem Support) mit konzentriertem Edukt kann nämlich ein Reaktionsumsatzmaximum erreicht werden, welches auch mit einer maximalen Nebenproduktbildung (Hochsieder) verbunden ist. Übersteigt der Anteil der hochsiedenen Nebenprodukte in Abhängigkeit von den Betriebsbedingungen (Druck und Temperatur) einen gewissen Wert, kann dies aufgrund der von der vorliegenden Mischung abhängigen Dampfdrücke der Einzelkomponenten in der Ausbildung einer Flüssigphase resultieren, die das Katalysatorsystem schädigen, blockieren oder auswaschen kann.

Die Aktivierung des Katalysatorsystems wird erfindungsgemäß vorzugsweise mit einer zeitlich verlängerten Umsatzsteigerung realisiert. So lässt sich für jede Kombination aus Druck, Temperatur und Zusammensetzung des Einsatzgemisches ein maximal zulässiger Umsatz für die Bildung von Nebenprodukten berechnen, der nicht überschritten werden darf, um die vorgenannten Probleme nicht entstehen zu lassen. Der Umsatz für die Bildung von Nebenprodukten kann auch in Abhängigkeit des Umsatzes für die Bildung der Produktaldehyde (= in Abhängigkeit der Aldehydkonzentration) ermittelt werden, d. h. die Anfahrprozedur richtet sich nach dem maximalen Umsatz der Einsatzolefine.

Bei bekannten Langzeit-Betriebsbedingungen des zweiten Reaktors, die einen zuverlässigen Umsatzgrad der Einsatzolefine von 20 bis 95%, vorzugsweise von 80% - 95% ermöglichen, kann die Anfahrprozedur so realisiert werden, dass die Zusammensetzung des Einsatzgemisches, welches in den zweiten Reaktor gefahren wird, stufenweise geändert wird, ohne dass der maximale Umsatz der Einsatzolefine überschritten wird.

Dabei kann die Zusammensetzung des Einsatzgemisches, die bei Langzeit-Betriebsbedingungen für einen zuverlässigen Umsatz der Olefine sorgt, so variiert werden dass bei konstantem Volumenstrom der Olefin- und/oder der Synthesegasanteil in mindestens zwei, vorzugsweise mehr als drei Stufen, insbesondere vier oder mehr Stufen, angehoben wird, ohne dass der maximale Umsatz der Einsatzolefine überschritten wird. Dem technischen Einsatzgemisch und Synthesegasgemisch können dazu in der oder den ersten Stufe(n) Inertgase wie z.B. N₂, Argon, Helium oder ähnliches zugeführt wird.

Mit fortschreitender Betriebszeit kann die Aktivität des Katalysators abnehmen, beispielsweise durch die Anreicherung von Hochsiedern und/oder der Belegung oder Desaktivierung von aktiven Zentren. Die Hochsieder können zu einer verstärkten Kondensation in den Poren führen, sodass die Poren nicht mehr oder langsamer für die Eduktolefine zugänglich sind. Andererseits können einige Nebenprodukte zu einem Zerfall des Katalysatorsystems führen, wodurch die Aktivität des Katalysators ebenfalls abnimmt. Eine Abnahme der Katalysatoraktivität kann beispielsweise anhand des Absinkens von Umsätzen oder Selektivitäten, insbesondere durch eine entsprechende Analytik mittels Ramanspektroskopie, Gaschromatographie oder Massendurchflussmesser (MDM) ermittelt werden. Möglich wäre auch ein modellbasiertes Überwachen der Katalysatoraktivität. Dies würde eine von den Betriebsbedingungen unabhängige Methode zur Überwachung der Katalysatoraktivität darstellen, aber auch um den Verlauf zu extrapolieren und damit eine Revisions-/Regenerationsplanung zu unterstützen.

In dem Fall einer unzureichenden Katalysatoraktivität kann das Katalysatorsystem, welches heterogenisiert auf dem porösen keramischen Support vorliegt, ausgetauscht werden. Dazu kann der Reaktor bzw. der Support in dem Reaktor einmalig oder mehrmalig mit einem Lösemittel gespült werden. Durch das Spülen kann das Katalysatorsystem demobilisiert und entfernt werden. Das Lösemittel kann eines der für die Herstellung der Katalysatorlösung genannten Lösemittel sein. Die Temperatur beim Spülen mit Lösemittel kann 20 bis 150 °C betragen. Der Druck kann beim Spülen mit Lösemittel zudem 1 bis 25 bar betragen.

Nach dem Spülen wird der Support ein- oder mehrfach neu imprägniert, insbesondere mit der vorher beschriebenen in-situ-Imprägnierung des Supports. Die in-situ-Imprägnierung wird damit erneuert und das heterogenisierte Katalysatorsystem frisch aufgebracht. Die erneute in-situ-Imprägnierung kann unter genau den gleichen Bedingungen durchgeführt werden, wie sie für die erste in-situ-Imprägnierung oben beschrieben worden sind.

Aufgrund der Tatsache, dass das Katalysatorsystem durch Spülen und erneutes Aufbringen vollständig ausgetauscht wird, können diese Schritte ständig wiederholt werden, sobald die Aktivität des Katalysators erneut absinkt. Ein weiterer Vorteil ist, dass sowohl Hochsieder und Produktaldehyde als auch Zerfallsprodukte des Katalysatorsystems ausgeschleust werden können. Es sollte jedoch darauf geachtet werden, dass die Eigenschaften des Supports durch Demobilisierung und erneute in-situ-Imprägnierung nicht beeinträchtigt werden. Andernfalls wäre auch ein Austausch des porösen keramischen Supports durchzuführen.

Eine weitere Option ist, dass der gesamte poröse keramische Support, auf dem das Katalysatorsystem heterogenisiert vorliegt, ausgetauscht wird. Das Katalysatorsystem, welches auf dem (aus dem Reaktor entfernten) Support heterogenisierten vorliegt, kann dann außerhalb des Reaktors wie oben beschrieben ausgetauscht und bis zum nächsten Einbau und Einsatz im Reaktor gelagert werden. Wie erwähnt ist beim Auftrag des Katalysatorsystems eine inerte Umgebung erforderlich, weshalb die Handhabung und Lagerung bei der genannten Verfahrensweise von Aus- und Einbau des Supports unter entsprechenden Bedingungen erfolgen sollte.

Grundsätzlich ist eine Verfahrensführung mit mehreren Reaktoren denkbar, bei der die Reaktoren parallel oder in Reihe geschaltet vorliegen. Bevorzugt ist eine Verfahrensführung, bei der in der zweiten Reaktionszone mehrere Reaktoren parallel geschaltet vorliegen und diese alternierend verwendet werden. Dabei wird mindestens ein Reaktor (a) für die Hydroformylierung eingesetzt, also einer, der in Betrieb ist, und mindestens ein Reaktor (b), der in Wartehaltung ist. Das ist so zu verstehen, dass sobald bei dem sich in Betrieb befindende Reaktor (a) eine nicht mehr ausreichende Katalysatoraktivität festgestellt wird, der Strom des zweiten Einsatzgemisches von diesem Reaktor (a) auf den nächsten Reaktor (b) in Wartehaltung, der damit in Betrieb genommen wird, umgeschaltet wird. Reaktor (a) wird dann in Regenerationsmodus überführt, wo das Katalysatorsystem wie oben beschrieben regeneriert oder demobilisiert und der Support neu imprägniert wird, und dann in die Warteposition überführt bis ein erneuter Wechsel mit Reaktor (b) erfolgt. Dies Prinzip lässt sich auch auf 3 oder mehr Reaktoren anwenden, wobei ein Reaktor in Betrieb ist, einer oder mehre Reaktoren gleichzeitig in Wartehaltung sind und einer oder mehrere Reaktoren gleichzeitig im Regenerationsmodus sind.

Der zweite Hydroformylierungsschritt wird vorzugsweise bei den folgenden Bedingungen durchgeführt: Die Temperatur beim zweiten Hydroformylierungsschritt sollte im Bereich von 65 bis 200 °C, vorzugsweise 75 bis 175 °C und besonders bevorzugt 85 bis 150 °C liegen. Der Druck sollte 35 bar, vorzugsweise 30 bar, besonders bevorzugt 25 bar während des zweiten Hydroformylierungsschritts nicht überschreiten. Das molare Verhältnis zwischen Synthesegas und dem zweiten Einsatzgemisch sollte zwischen 6:1 und 1:1 liegen. Optional kann das zweite Einsatzgemisch mit Inertgas verdünnt werden, beispielsweise den in technischen Kohlenwasserstoffströmen befindlichen Alkanen.

In einer Ausführungsform der vorliegenden Erfindung ist der zweite Hydroformylierungsschritt nach, also stromabwärts, der ersten und zweiten Stofftrennung angeordnet, wobei der zweite Hydroformylierungsschritt mit der an nicht umgesetzten Olefinen-reichen Phase als zweitem Einsatzgemisch gespeist wird. Die bei der Stofftrennung gebildete Produktaldehyd-reiche Phase kann in diesem Fall der nachfolgenden Aldehydabtrennung zugeführt werden, bei der der Produktaldehyd von den genannten in dieser Phase befindlichen Stoffen abgetrennt wird. Der Purgegasstrom kann ebenfalls zur zweiten Stofftrennung geführt werden, um die darin enthaltenen Produktaldehyde abzutrennen und um Verunreinigungen (z. B. Stickstoff im Synthesegas) oder inerte Substanzen (z. B. Alkane im Einsatzgemisch) nachfolgend aus dem System auszutragen. Die Verunreinigungen oder inerte Substanzen können üblicherweise bei der zweiten Stofftrennung als leichtflüchtige Stoffe, beispielsweise am Kopf einer Kolonne, abgenommen werden

Der erhaltene Austrag aus der zweiten Hydroformylierung kann danach über eine dritte Stofftrennung in mindestens eine zweite an nicht umgesetzten Olefin reiche Phase und mindestens eine zweite Produktaldehyd-reiche Phase getrennt wird. Die zweite an nicht umgesetzten Olefinreiche Phase kann zur zweiten Stofftrennung oder zur zweiten Reaktionszone zurückgeführt werden (recycliert), um die darin enthaltenen Produktaldehyde abzutrennen und/oder nicht umgesetzte Olefine noch zu hydroformylieren. Die bei der dritten Stofftrennung gebildete zweite Produktaldehyd-reiche Phase kann optional zusammen mit der bei der zweiten Stofftrennung gebildeten ersten Produktaldehyd-reichen Phase einer nachfolgenden Aldehydabtrennung zugeführt werden, bei der das Produktaldehyd von den anderen in dieser Phase befindlichen Stoffen getrennt wird. Andererseits ist es auch möglich, dass bei der zweiten und/oder dritten Stofftrennung bereits ein so reiner Produktaldehyd abgetrennt wird, dass keine weitere Aldehydabtrennung erforderlich ist.

Die Anlage, in der das vorliegende Verfahren durchgeführt werden kann, umfasst einen ersten Reaktor, in dem der erste Hydroformylierungsschritt durchgeführt wird, und einen zweiten Reaktor, in dem der zweite Hydroformylierungsschritt durchgeführt wird. Zusätzlich erfasst die Anlage zwei Stofftrennungseinheiten zwischen dem ersten und dem zweiten Reaktor. Nach dem zweiten Reaktor folgt stromabwärts eine dritte Stofftrennungseinheit, mit der der Produktaldehyd aus dem zweiten Hydrofromylierungsschritt abgetrennt wird.

Die Erfindung wird anhand der folgenden Abbildungen, die die beschriebenen Ausführungsformen exemplarisch zeigen, erläutert. Diese Abbildungen sind lediglich eine Veranschaulichung der Erfindung und daher nicht einschränkend zu verstehen.
- Abbildung 1:: Vereinfachtes Verfahrensfließbild des erfindungsgemäßen Verfahrens mit zwei Stofftrennungsschritten zwischen dem ersten und dem zweiten Reaktor, wobei der Purgegasstrom sowohl zur zweiten Stofftrennung und zum zweiten Reaktor geführt werden kann.
- Abbildung 2:: Vereinfachtes Verfahrensfließbild des erfindungsgemäßen Verfahrens mit zwei Stofftrennungsschritten zwischen dem ersten und dem zweiten Reaktor, wobei der Purgegasstrom zur zweiten Stofftrennung geführt werden kann.

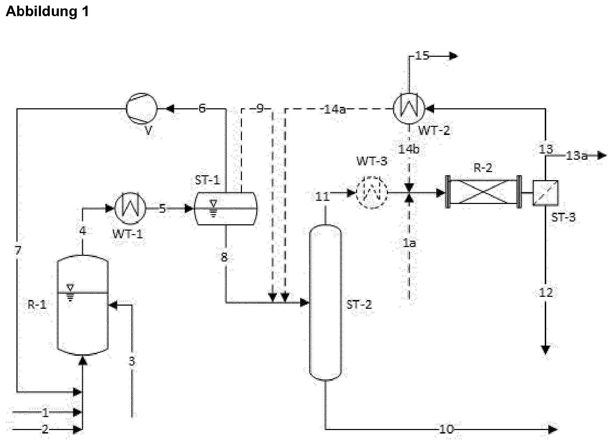

Abbildung 1 zeigt ein vereinfachtes Verfahrensfließbild zur Durchführung einer Ausführungsform des erfindungsgemäßen Hydroformylierungsverfahrens. Der dafür notwendige Anlagenkomplex setzt sich wie folgt zusammen. Gestrichelte Linien deuten optionale Ausgestaltungen an.

Die Anlage umfasst eine erste Reaktionszone, die aus dem ersten Reaktor R-1 besteht, die mit Synthesegas über die Leitung 1, dem Einsatzgemisch, umfassend die C2- bis C5-Olefine, über die Leitung 2 und dem homogenen Katalysatorsystem über Leitung 3 (soweit im laufenden Betrieb erforderlich) gespeist wird. In der ersten Reaktionszone wird der erste Hydroformylierungsschritt, also die Hydroformylierung der Einsatzolefine zu den entsprechenden Aldehyden, des erfindungsgemäßen Verfahrens durchgeführt. Innerhalb des Reaktors R-1 der primären Reaktionszone bildet sich dabei üblicherweise eine flüssige Reaktionsphase aus, die innerhalb des Reaktors R-1 in der Abbildung angedeutet ist. Die flüssige Phase enthält dabei hauptsächlich das flüssige Produktaldehyd, gelöstes Synthesegas die Einsatzolefine und das gelöste Katalysatorsystem.

Aus dem ersten Reaktor R-1 wird ein gasförmiger Austrag, der sowohl Produktaldehyde als auch nicht umgesetzte Olefine und Synthesegas umfasst, über die Leitung 4 abgezogen. Das Abziehen des gasförmigen Austrags kann durch den Verdichter V gesteuert werden. Der gasförmige Austrag wird über Leitung 4 zu einem Wärmetauscher WT-1 und dann über Leitung 5 zur ersten Stofftrennung ST-1, insbesondere einem Kondensator, geführt. Bei der ersten Stofftrennung ST-1 fallen - wie oben beschrieben - eine Synthesegas-reiche Phase und eine Synthesegas-arme Phase an. Die Synthesegas-reiche Phase wird über die Leitung 6 zum Verdichter V und von dort über Leitung 7 zum Reaktor R-1 zurückgeführt.

Die Synthesegas-arme Phase wird über Leitung 8 zur zweiten Stofftrennung ST-2, insbesondere einer Destillationskolonne, geführt. Ein eventueller Purgegasstrom kann in einer optionalen Ausgestaltung über Leitung 9 ebenfalls zur zweiten Stofftrennung ST-2 geführt werden, um die darin enthaltenen Produktaldehyde abzutrennen.

Bei der zweiten Stofftrennung ST-2 fallen - wie oben beschrieben - eine an nicht umgesetzten Olefinen-reiche Phase und eine Produktaldehyd-reiche Phase an. Die Produktaldehyd-reiche Phase wird über Leitung 10 der weiteren Aufarbeitung zugeführt. Die an nicht umgesetzten Olefinen-reiche Phase wird über die Leitung 11 und einen optionalen Wärmetauscher WT-3 zum zweiten Reaktor R-2 geführt. Falls nötig kann dem Reaktor R-2 zusätzlich Synthesegas über die Leitung 1a zugeführt werden.

Im Reaktor R-2 wird der zweite Hydroformylierungsschritt durchgeführt, wonach eine zweite Stofftrennung ST-3 in zumindest eine Produktaldehyd-reiche Phase und eine Produktaldehyd-abgereicherte Phase stattfindet. Die an Produktaldehyd-abgereicherte Phase wird über die Leitungen 13 und 14 zur zweiten Stofftrennung ST-2 und/oder zum zweiten Reaktor R-2 zurückgeführt. Dabei wird ein Wärmetauscher WT-2 passiert, wo nachfolgend über die Leitung 15 Synthesegas und inerte Stoffe ausgeschleust werden können. Alkane können auch über die Leitung 13a abgenommen werden. Die in der dritten Stofftrennung ST-3 gebildete zweite Produktaldehyd-reiche Phase wird über Leitung 12 der weiteren Aufarbeitung zugeführt, gegebenenfalls auch kombiniert mit der ersten Produktaldehyd-reichen Phase (Leitung 10).

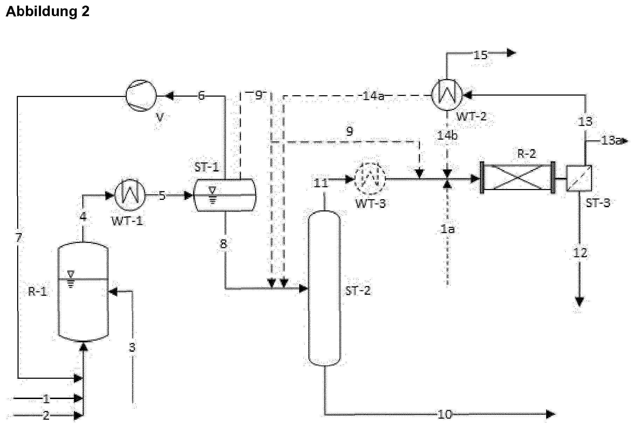

Abbildung 2 zeigt eine weitere Ausführungsform der vorliegenden Erfindung, bei der optionale Purgegasstrom über Leitung 9 sowohl zur zweiten Stofftrennung ST-2 als auch zum zweiten Reaktor R-2 geführt wird, um die darin enthaltenen Produktaldehyde abzutrennen und nicht umgesetzte Olefine zu hydroformylieren. Das restliche Verfahren ist mit dem in Abbildung 1 beschriebenen Verfahren identisch.

Nachfolgend wird die vorliegende Erfindung anhand von Beispielen näher erläutert. Alternative Ausführungsformen der vorliegenden Erfindung sind in analoger Weise erhältlich.

### Beispiel:

### Versuch 1: Verwendung eines erfindungsgemäßen Katalysatorsystems

Als Support wurde ein Monolith aus Siliciumcarbid mit einer Länge von ca. 20 cm und einem Durchmesser von ca. 25 mm verwendet. Der Support war porös und wurde mit einem Washcoat (SiOz) vorbehandelt. Der Support wies 31 Kanäle mit einem Durchmesser von ca. 3 mm auf. Der Support wurde in einen Reaktor eingesetzt und mit einer Katalysatorlösung, enthaltend Rh(acac)(CO)₂, Bisphephos (Ligand), Bis(2,2,6,6-tetramethyl-4-piperidyl)sebacat (Stabilisator), 1-Ethyl-3-methylimidazolium-bis(trifluoromethansulfonyl)imid ([EMIM][NTf₂] / ionische Flüssigkeit) und Dichlormethan als Lösemittel und hergestellt durch Mischen in einer inerten Umgebung (Glovebox), beaufschlagt. Dazu wurde die Katalysatorlösung nach dem Spülen des Reaktors mit Stickstoff mit leichtem Überdruck in den Reaktor eingeleitet. Nach Entfernen des Lösemittels aus dem Reaktor durch Ablassen und Verdampfen wurde das auf dem Support heterogenisierte Katalysatorsystem zur Hydroformylierung verwendet.

Als Einsatzgemisch wurde ein Kohlenwasserstoffstrom mit der folgenden Zusammensetzung verwendet:

| | Menge (Gew.-%) |
|---|---|
| 1-Buten / iso-Buten | 19,14 |
| cis-2-Buten | 19,10 |
| trans-2-Buten | 28,40 |
| n-Butan | 30,80 |
| iso-Butan | 0,02 |
| 2-Methyl-butan | 2,50 |

Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 °C und einem Druck von 10 bar durchgeführt. Der Gesamtumsatz an Butenen (also der Umsatz aller im Einsatzgemisch befindlichen Butene) und die n/iso-Selektivität (Verhältnis von linearen zu verzweigten Produkten) wurde gaschromatographisch über die Produktzusammensetzung ermittelt.

Nach einer Versuchsdauer von 500 Stunden betrug der Gesamtumsatz an Butenen 27% und die n/iso-Selektivität 98%.

### Versuch 2: Verwendung eines erfindungsgemäßen Katalysatorsystems ohne ionische Flüssigkeit

Als Support wurde ein Monolith aus Siliciumcarbid mit einer Länge von ca. 20 cm und einem Durchmesser von ca. 25 mm verwendet. Der Support war porös und wurde mit einem Washcoat (SiOz) vorbehandelt. Der Support wies 31 Kanäle mit einem Durchmesser von ca. 3 mm auf. Der Support wurde in einen Reaktor eingesetzt und mit einer Katalysatorlösung, enthaltend Rh(acac)(CO)₂, Biphephos (Ligand), Bis(2,2,6,6-tetramethyl-4-piperidyl)sebacat (Stabilisator) und Dichlormethan als Lösemittel und hergestellt durch Mischen in einer inerten Umgebung (Glovebox), beaufschlagt. Dazu wurde die Katalysatorlösung nach dem Spülen des Reaktors mit Stickstoff mit leichtem Überdruck in den Reaktor eingeleitet. Nach Entfernen des Lösemittels aus dem Reaktor durch Ablassen und Verdampfen wurde das auf dem Support heterogenisierte Katalysatorsystem zur Hydroformylierung verwendet.

Als Einsatzgemisch wurde ein Kohlenwasserstoffstrom mit nahezu identischer Zusammensetzung wie in Versuch 1 verwendet. Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 °C und einem Druck von 10 bar durchgeführt. Der Gesamtumsatz an Butenen (also der Umsatz aller im Einsatzgemisch befindlichen Butene) und die n/iso-Selektivität (Verhältnis von linearen zu verzweigten Produkten) wurde gaschromatographisch über die Produktzusammensetzung ermittelt.

Nach einer Versuchsdauer von 500 Stunden betrug der Gesamtumsatz an Butenen 56% und die n/iso-Selektivität 97%.

### Versuch 3: Verwendung eines nicht erfindungsgemäßen SILP-Katalysatorsystems

Die Herstellung des Katalysatorsystems erfolgte analog zu der Herstellung der katalytisch aktiven Zusammensetzung Rh(II) in der WO 2015/028284 A1.

Als Einsatzgemisch wurde ein Kohlenwasserstoffstrom mit der folgenden Zusammensetzung verwendet:

| | Menge (Gew.-%) |
|---|---|
| 1-Buten / iso-Buten | 27,40 |
| cis-2-Buten | 15,00 |
| trans-2-Buten | 25,00 |
| n-Butan | 29,50 |
| iso-Butan | 0,02 |
| 2-Methylbutan | 3,00 |

Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 °C und einem Druck von 10 bar durchgeführt. Der Gesamtumsatz an Butenen (also der Umsatz aller im Einsatzgemisch befindlichen Butene) und die n/iso-Selektivität (Verhältnis von linearen zu verzweigten Produkten) wurde gaschromatographisch über die Produktzusammensetzung ermittelt.

Nach einer Versuchsdauer von 500 Stunden betrug der Gesamtumsatz an Butenen 25% und die n/iso-Selektivität 92%.

Aus den Versuchsreihen ist somit zu erkennen, dass die erfindungsgemäßen heterogenisierten Katalysatorsystemen gegenüber den bekannten SILP-Systemen den Vorteil haben, dass damit höhere Umsätze und höheren Linearität der Produkte (n/iso-Selektivität) erreicht werden können.

## Patentansprüche

1. Verfahren zur Herstellung von Aldehyden durch Hydroformylierung eines ersten Einsatzgemisches, welches C2- bis C5-Olefine als Edukte umfasst, wobei die Hydroformylierung mindestens zwei Hydroformylierungsschritte umfasst, **dadurch gekennzeichnet, dass**
in dem ersten Hydroformylierungsschritt das Einsatzgemisch mit Synthesegas in Gegenwart eines ersten Katalysatorsystems in einer ersten Reaktionszone hydroformyliert wird,
aus der ersten Reaktionszone kontinuierlich ein gasförmiger Austrag entnommen wird, der mindestens einen Teil der entstandenen Produktaldehyde, mindestens einen Teil der nicht umgesetzten Olefine und Synthesegas enthält,
der gasförmige Austrag aus der ersten Hydroformylierung einem ersten Stofftrennungsschritt unterworfen wird, bei der der gasförmige Austrag in eine Synthesegas-reiche Phase, in eine Synthesegas-arme-Phase und optional einen Purgegasstrom getrennt wird, wobei die Synthesegas-reiche Phase zur ersten Reaktionszone zurückgeführt wird und die Synthesegas-arme Phase einem zweiten Stofftrennungsschritt unterworfen wird, bei der die Synthesegas-arme Phase in mindestens eine an nicht umgesetzten Olefinen-reiche Phase und mindestens eine Produktaldehyd-reiche Phase getrennt wird;
die nach Durchlaufen des zweiten Stofftrennungsschritts erhaltene an nicht umgesetzten Olefinen-reiche Phase und optional zumindest ein Teil des Purgegasstroms als zweites Einsatzgemisch zum zweiten Hydroformylierungsschritt geleitet wird; und
in dem zweiten Hydroformylierungsschritt ein zweites Einsatzgemisch in gasförmigem Zustand mit Synthesegas in Gegenwart eines zweiten Katalysatorsystems, welches ein Metall aus der 8. oder 9. Gruppe des Periodensystems der Elemente, mindestens einen organischen Phosphor-enthaltenden Liganden, einen Stabilisator und optional eine ionische Flüssigkeit umfasst, in einer zweiten Reaktionszone einer weiteren Hydroformylierung unterworfen wird, wobei das zweite Einsatzgemisch und das Synthesegas über einen Support aus einem porösen keramischen Material geleitet werden, auf dem das Katalysatorsystem heterogenisiert vorliegt,
wobei der Support ein Monolith, das heißt ein Block aus einem keramischen Material ist, auf den ein Washcoat aus, bezogen auf das keramische Material des Supports, dem gleichen oder einem anderen keramischen Material aufgetragen wird.

2. Verfahren nach Anspruch 1, wobei der organische Phosphor-enthaltende Ligand des Hydroformylierungskatalysatorsystems die allgemeine Formel (VI)
R'-A-R"-A-R‴ (VI)
aufweist, wobei R', R" und R‴ jeweils organische Reste sind, mit der Maßgabe das R' und R‴ nicht identisch sind, und beide Ajeweils eine brückende -O-P(-O)₂-Gruppe sind, wobei zwei der drei Sauerstoffatome -O-jeweils an Rest R' und den Rest R‴ gebunden sind.

3. Verfahren nach Anspruch 1 oder 2, wobei der Stabilisator eine organische Aminverbindung ist, die mindestens eine 2,2,6,6-Tetramethylpiperidineinheit nach Formel (I) enthält:

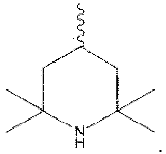

4. Verfahren nach einem der Ansprüche 1 bis 3, wobei das poröse keramische Material, aus dem der Support besteht, aus der Gruppe, bestehend aus einer Silikatkeramik, einer oxidischen Keramik, einer nitridischen Keramik, einer carbidischen Keramik, einer silicidischen Keramik und Mischungen davon, ausgewählt wird.

5. Verfahren nach Anspruch 4, wobei die Silikatkeramik ausgewählt wird aus Alumosilikat, Magnesiumsilikat und Mischungen davon, wie z. B. Bentonit; die oxidische Keramik ausgewählt wird aus γ-Aluminiumoxid, α-Aluminiumoxid, Titandioxid, Beryliumoxid, Zirkoniumoxid, Aluminiumtitanat, Bariumtitanat, Zinkoxid, Eisenoxiden (Ferrite) und Mischungen davon; die nitridische Keramik ausgewählt wird aus Siliciumnitrid, Bornitrid, Aluminiumnitrid und Mischungen davon; die carbidische Keramik ausgewählt wird aus Siliciumcarbid, Borcarbid, Wolframcarbid oder Mischungen davon; und die silicidische Keramik Molbydänsilicid ist.

6. Verfahren nach Anspruch 4, wobei das poröse keramische Material, aus dem der Support besteht, eine carbidische Keramik ist.

7. Verfahren nach Anspruch 6, wobei die carbidische Keramik ausgewählt wird aus Siliciumcarbid, Borcarbid, Wolframcarbid oder Mischungen davon.

8. Verfahren nach einem der Ansprüche 1 bis 7, wobei die Menge das auf dem Support befindlichen Waschcoats ≤ 20 Gew.-% beträgt, bezogen auf die Gesamtmenge des Supports

9. Verfahren nach einem der Ansprüche 1 bis 8, wobei der Support aus dem keramischen Material einen oder mehrere Kanäle in Hauptdurchströmungsrichtung aufweist.

10. Verfahren nach einem der Ansprüche 1 bis 9, wobei der zweiten Hydroformylierungsschritt bei einer Temperatur im Bereich von 65 bis 200 °C durchgeführt wird.

11. Verfahren nach einem der Ansprüche 1 bis 10, wobei der Druck im zweiten Hydroformylierungsschritt nicht größer als 35 bar ist.

## Claims

1. Process for producing aldehydes by hydroformylation of a first feed mixture comprising C2- to C5-olefins as starting materials, wherein the hydroformylation comprises at least two hydroformylation steps, **characterized in that**
in the first hydroformylation step, the feed mixture is hydroformylated with synthesis gas in the presence of a first catalyst system in a first reaction zone,
a gaseous discharge is withdrawn continuously from the first reaction zone comprising at least a portion of the product aldehydes formed, at least a portion of the unreacted olefins and synthesis gas,
the gaseous discharge from the first hydroformylation is subjected to a first substance separation step in which the gaseous discharge is separated into a synthesis gas-rich phase, a synthesis gas-depleted phase and optionally a purge gas stream, wherein the synthesis gas-rich phase is recycled to the first reaction zone and the synthesis gas-depleted phase is subjected to a second substance separation step in which the synthesis gas-depleted phase is separated into at least one unreacted olefin-rich phase and at least one product aldehyde-rich phase;
the unreacted olefin-rich phase obtained after undergoing the second substance separation step and optionally at least a portion of the purge gas stream is conducted as second feed mixture to the second hydroformylation step; and
in the second hydroformylation step, a second feed mixture in the gaseous state is subjected to a further hydroformylation with synthesis gas in a second reaction zone in the presence of a second catalyst system comprising a metal from Group 8 or 9 of the Periodic Table of the Elements, at least one organic phosphorus-containing ligand, a stabilizer and optionally an ionic liquid, wherein the second feed mixture and the synthesis gas are passed over a support composed of a porous ceramic material on which the catalyst system is in heterogenized form,
wherein the support is a monolith, i.e. a block composed of a ceramic material on which a washcoat composed of the same or another ceramic material, based on the ceramic material of the support, is applied.

2. Process according to Claim 1, wherein the organic phosphorus-containing ligand of the hydroformylation catalyst system has the general formula (VI)
R'-A-R"-A-R‴ (VI)
where R', R" and R‴ are each organic radicals, with the proviso that R' and R‴ are not identical, and each A is a bridging -O-P(-O)₂ group, wherein two of the three oxygen atoms -O- are each bonded to radical R' and the radical R‴.

3. Process according to Claim 1 or 2, wherein the stabilizer is an organic amine compound comprising at least one 2,2,6,6-tetramethylpiperidine unit according to formula (I):

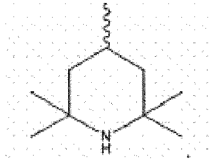

4. Process according to any of Claims 1 to 3, wherein the porous ceramic material of which the support consists is selected from the group consisting of a silicate ceramic, an oxidic ceramic, a nitridic ceramic, a carbidic ceramic, a silicidic ceramic and mixtures thereof.

5. Process according to Claim 4, wherein the silicate ceramic is selected from aluminosilicate, magnesium silicate and mixtures thereof, for example bentonite; the oxidic ceramic is selected from γ-alumina, α-alumina, titanium dioxide, beryllium oxide, zirconium oxide, aluminium titanate, barium titanate, zinc oxide, iron oxides (ferrites) and mixtures thereof; the nitridic ceramic is selected from silicon nitride, boron nitride, aluminium nitride and mixtures thereof; the carbidic ceramic is selected from silicon carbide, boron carbide, tungsten carbide or mixtures thereof; and the silicidic ceramic is molybdenum silicide.

6. Process according to Claim 4, wherein the porous ceramic material of which the support consists is a carbidic ceramic.

7. Process according to Claim 6, wherein the carbidic ceramic is selected from silicon carbide, boron carbide, tungsten carbide or mixtures thereof.

8. Process according to any of Claims 1 to 7, wherein the amount of washcoat on the support is ≤ 20% by weight, based on the total amount of the support.

9. Process according to any of Claims 1 to 8, wherein the support composed of the ceramic material has one or more channels in the main direction of flow through.

10. Process according to any of Claims 1 to 9, wherein the second hydroformylation step is carried out at a temperature in the range of 65 to 200°C.

11. Process according to any of Claims 1 to 10, wherein the pressure in the second hydroformylation step is not greater than 35 bar.

## Revendications

1. Procédé pour la préparation d'aldéhydes par hydroformylation d'un premier mélange de départ, qui comprend des oléfines en C2-C5 comme produits de départ, l'hydroformylation comprenant au moins deux étapes d'hydroformylation, **caractérisé en ce que** dans la première étape d'hydroformylation, le mélange de départ est hydroformylé avec un gaz de synthèse en présence d'un premier système catalytique dans une première zone de réaction,
un produit évacué gazeux est prélevé en continu de la première zone de réaction, lequel produit contient au moins une partie des aldéhydes produits formés, au moins une partie des oléfines non transformées et du gaz de synthèse,
le produit évacué gazeux provenant de la première hydroformylation est soumis à une première étape de séparation de substances, dans laquelle le produit évacué gazeux est séparé en une phase riche en gaz de synthèse, en une phase pauvre en gaz de synthèse et éventuellement en un flux de gaz de purge, la phase riche en gaz de synthèse étant recyclée vers la première zone de réaction et la phase pauvre en gaz de synthèse étant soumise à une deuxième étape de séparation de substances, dans laquelle la phase pauvre en gaz de synthèse est séparée en au moins une phase riche en oléfines non transformées et en au moins une phase riche en aldéhydes produits ;
la phase riche en oléfines non transformées obtenue après le passage dans la deuxième étape de séparation de substances et éventuellement au moins une partie du flux de gaz de purge sont guidés en tant que deuxième mélange de départ vers la deuxième étape d'hydroformylation ; dans la deuxième étape d'hydroformylation, un deuxième mélange de départ est soumis à l'état gazeux avec du gaz de synthèse, en présence d'un deuxième système catalytique, qui comprend un métal du 8ème ou du 9ème groupe du système périodique des éléments, au moins un ligand organique contenant du phosphore, un stabilisant et éventuellement un liquide ionique, dans une deuxième zone de réaction à une autre hydroformylation, le deuxième mélange de départ et le gaz de synthèse étant guidés sur un support constitué par un matériau céramique poreux, sur lequel le système catalytique se trouve sous forme hétérogène,
le support étant un monolithe, c'est-à-dire un bloc en matériau céramique sur lequel une couche d'imprégnation constituée par le même ou un autre matériau céramique, par rapport au matériau céramique du support, est appliquée.

2. Procédé selon la revendication 1, le ligand organique contenant du phosphore du système catalytique d'hydroformylation présentant la formule générale (VI)
R'-A-R"-A-R‴ (VI)
dans laquelle R', R" et R"' représentent à chaque fois des radicaux organiques, à condition que R' et R"' ne soient pas identiques et les deux A représentent à chaque fois un groupe -O-P(-O)₂- formant un pont, deux des trois atomes d'oxygène -O- étant respectivement liés au radical R' et au radical R"'.

3. Procédé selon la revendication 1 ou 2, le stabilisant étant un composé organique d'amine, qui contient au moins un motif 2,2,6,6-tétraméthylpipéridine selon la formule (I) :

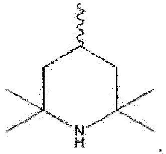

4. Procédé selon l'une quelconque des revendications 1 à 3, le matériau céramique poreux, dont est constitué le support, étant choisi dans le groupe constitué par une céramique de silicate, une céramique oxyde, une céramique de nitrure, une céramique de carbure, une céramique de siliciure et leurs mélanges.

5. Procédé selon la revendication 4, la céramique de silicate étant choisie parmi l'aluminosilicate, le silicate de magnésium et leurs mélanges, comme par exemple la bentonite ; la céramique oxyde étant choisie parmi le γ-oxyde d'aluminium, l'α-oxyde d'aluminium, le dioxyde de titane, l'oxyde de béryllium, l'oxyde de zirconium, le titanate d'aluminium, le titanate de baryum, l'oxyde de zinc, les oxydes de fer (ferrites) et leurs mélanges ; la céramique de nitrure étant choisie parmi le nitrure de silicium, le nitrure de bore, le nitrure d'aluminium et leurs mélanges ; la céramique de carbure étant choisie parmi le carbure de silicium, le carbure de bore, le carbure de tungstène ou leurs mélanges ; et la céramique de siliciure étant le siliciure de molybdène.

6. Procédé selon la revendication 4, le matériau céramique poreux, dont est constitué le support, étant une céramique de carbure.

7. Procédé selon la revendication 6, la céramique de carbure étant choisie parmi le carbure de silicium, le carbure de bore, le carbure de tungstène ou leurs mélanges.

8. Procédé selon l'une quelconque des revendications 1 à 7, la quantité de couche d'imprégnation se trouvant sur le support étant ≤ 20% en poids, par rapport à la quantité totale du support.

9. Procédé selon l'une quelconque des revendications 1 à 8, le support en matériau céramique présentant un ou plusieurs canaux dans la direction d'écoulement principale.

10. Procédé selon l'une quelconque des revendications 1 à 9, la deuxième étape d'hydroformylation étant réalisée à une température dans la plage de 65 à 200°C.

11. Procédé selon l'une quelconque des revendications 1 à 10, la pression dans la deuxième étape d'hydroformylation n'étant pas supérieure à 35 bars.
